# EUROPEAN PATENT APPLICATION

(11) **EP 2 345 423 A1**
(43) Date of publication of application: **20.07.2011**
(21) Application number: 09755922.3
(22) Date of filing: 06.10.2009
(51) Int. Cl.: A61K 39/39, C12N 5/0783, A61K 35/14, A61K 38/19

(54) **ONCOSTATIN M AS PROMOTER OF IMMUNOSTIMULATORY ACTIVITY OF HUMAN EPITHELIAL CELLS**

(30) Priority: 07.10.2008 ES 200802835
(71) Applicant: Proyecto de Biomedicina Cima, S.L., 31008 Pamplona - Navarra (ES)
(72) Inventor: ALDABE ARREGUI, Rafael, E-31008 Pamplona - Navarra (ES); GONZÁLEZ DE LA TAJADA, Iranzu, E-31008 Pamplona - Navarra (ES); LARREA LEOZ, María Esther, E-31008 Pamplona - Navarra (ES); PRIETO VALTUEÑA, Jesús María, E-31008 Pamplona - Navarra (ES); SAROBE UGARRIZA, Pablo, E-31008 Pamplona - Navarra (ES)
(74) Representative: ABG Patentes, S.L.
(86) International application number: PCT/ES2009/070419
(87) International publication number: WO 2010/040882

(57) **Abstract**

The invention relates to the use of oncostatin M (OSM), an OSM receptor (OSMR) agonist, or a combination of OSM or a OSMR agonist with an interferon type I; in the manufacture of a medicament for enhancing the immunostimulatory activity of epithelial cells in a human.

## Description

### Field of the invention

The technical field of the invention is immunology.

### Background of the invention

Oncostatin M (OSM), a glycoprotein with an approximate molecular weight of 28,000, is an IL-6-type pleiotropic cytokine (IL-6 being the abbreviation for interleukin 6), produced mainly by T cells, monocytes/macrophages (Malik, 1989), and dendritic cells (Suda, et al., 2002). It was originally isolated from human leukemia cells and identified by its capacity to inhibit melanoma cell growth in vitro (Zarling, et al., 1986; Brown, et al., 1987). OSM promotes the production of IL-6, an important regulator of the host defense system (Brown, et al., 1991), as well as the expression of acute phase proteins (Heinrich, 1998). OSM is now considered as a multifunctional cytokine implicated in the activation, proliferation, and differentiation of several cell types, such as hepatocytes, osteoblasts, or lung epithelial cells (Grant, et al., 1999; Tanaka, et al., 2003), as well as a player in inflammatory responses.

Besides its growth inhibitory activities on human A375 melanoma and mouse M1 myeloid leukemic cells, as well as on other solid tumor cells (Grant, et al., 1999; Gibbs, et al., 1998), OSM also has growth stimulatory activities on normal fibroblasts, AIDS-Kaposi's sarcoma cells, and a human erythroleukemia cell line, TF-1 (Nair, et al., 1992; Miles, et al., 1992). The use of antibodies binding to OSM receptor beta subunit (OSMRβ) has been claimed for inhibiting cancer cell growth (WO2006084092).

OSM in combination with interferon-alpha is also known for its antiviral activities, particularly in inhibiting the replication of the Hepatitis C virus (EP1905447).

Zarling, et al., 1986, already established the role of OSM as an inflammatory mediator. However, its precise effect on the immune system as on other cytokines is not clearly understood. Moreover, the unknown differences between the OSM receptor-signaling systems in mice and humans prior to 1997 (Ichihara, et al., 1997) have added confusion to the characterization of the biological activities of OSM.

On the one hand, Brown, et al., 1991, described the pro-inflammatory properties of OSM on endothelial cells. Yao, et al., 1996 also reported that stimulation of a primary endothelial culture (HUVEC) with human OSM resulted in delayed but prolonged upregulation of P-selectin, which facilitates leukocyte emigration into sites of chronic or allergic inflammation. On the other hand, OSM was found to reduce the degree of joint destruction in an antibody induced model of rheumatoid arthritis, indicating an antiinflammatory activity without concordant immunosuppression (Wallace, et al., 1999). Hams, et al., 2008 concluded that OSMRβ signaling limited monocytic cell recruitment during acute inflammation. These last two studies, however, were performed in murine models.

In humans, OSM signals through two different receptor complexes: a) the LIF/OSM shared receptor (LIF being the abbreviation of leukemia inhibitory factor), which shares high-affinity binding with LIF-an evolutionary related protein with structural similarity to OSM; and b) the OSM-specific receptor, which binds OSM uniquely. However, in mice, OSM signals only through the murine homologue of the OSM-specific receptor. In addition, human OSM, which has been traditionally used in in vitro and in vivo studies in mice, binds only to the murine LIF receptors, thereby exhibiting only biological activities belonging to LIF but not to OSM (Bruce, et al., 2000). Thus with the aim of uncovering biological activities of OSM with a clinical application in humans, properly designed studies should employ human cells.

In EP422186, there is described the role of OSM in inhibiting endothelial tissue immunogenicity to helper and effector T lymphocytes, with applications in organ transplantation and the treatment of autoimmune diseases.

In Durda, et al., 2003, OSM is presented as a down-modulator of melanocyte antigen expression. However, no conclusive data is provided as to the effects of this diminished antigen expression on the immune response towards melanoma cells.

In an effort to characterize further the immunological properties of OSM in human models, it has been surprisingly found that OSM enhances the ability of human hepatocellular liver carcinoma (HepG2) cells to stimulate the production of interferon gamma (IFN-γ) by cytotoxic T cells (CTL) more efficiently than when using interferon alpha2 (IFNα2) alone. IFN-γ is a commonly used marker of T lymphocyte activation after antigen recognition in cell-mediated immunity (Brosterhus, et al., 1999). The present findings have in particular revealed that OSM increases the antigenicity of infected or tumoral epithelial cells, i.e. by increasing antigen processing and presentation in these cells, making these cells more visible and reactive to immune responses, in a suicidal type of mechanism that results in a selective destruction of the same cells.

Type I interferons are a family of polypeptides with cytokine activity which were originally discovered by virtue of their inhibitory activity on the viral infection of cells lines in vitro (Pestka, et al., 2004).

Depending on the homology of their sequences, type I interferons are classified into interferon-α (IFN-α), interferon-β (IFN-β) and interferon-ω (IFN-ω). The function of these cytokines is very important in the immune response against multiple types of viral infections, because they initiate mechanisms promoting the apoptosis-induced death of the infected cells and viral replication inhibition while favoring antigen presentation. It has recently been experimentally documented that they also carry out their functions by directly activating the activities of NK, B, and T-cells, as well as of dendritic cells in the immune response (Le Bon A et al., 2006; Le Bon A et al., 2006; Le Bon, et al., 2003)

Type I interferons have been tested in a variety of clinical disease states. IFN-α is prescribed in the treatment of chronic viral hepatitis and is used in the treatment of several malignant diseases, such as melanoma and chronic myeloid leukemia. For example, Murata et al., 2006, describe the anti-tumor effects of interferons α and β on hepatocellular carcinoma cell lines, such as an antiproliferative effect, change of cell cycle, and apoptosis. The anti-tumor effect of IFN-α is mediated by direct pro-apoptotic effects on tumor cells, anti-angiogenic effects on vascular tumor cells, and enhancing effects on the anti-tumor immune response. However, clinical trials show that the efficacy of IFN-α in the treatment of tumors is very limited, such that the clinical benefits compared to the adverse effects are not very favorable, and therefore its use in oncology has currently been very limited. The use of human interferon beta is best established in the treatment of multiple sclerosis.

In addition to the ongoing characterization of the immunological properties of OSM, it has also been surprisingly found that OSM synergizes with IFNα2 in enhancing the ability of human hepatocellular liver carcinoma (HepG2) cells to stimulate the production of IFN-γ by cytotoxic T cells. OSM alone or in combination with IFNs type I are therefore useful and potent agents in the immunotherapy of cancer and infectious diseases.

### Summary of the invention

The present invention relates to the use of oncostatin M (OSM), a OSM receptor (OSMR) agonist, or a combination of OSM or a OSMR agonist with a type I interferon; in the manufacture of a medicament for enhancing the immunostimulatory activity of epithelial cells in a human. Similarly, the present invention relates to OSM, a OSMR agonist, or a combination of OSM or a OSMR agonist with a type I IFN for use in enhancing the immunostimulatory activity of epithelial cells in a human. There is also provided a method of enhancing the immunostimulatory activity of epithelial cells in a human, comprising administering to said human an effective amount of OSM, a OSMR agonist, or a combination of OSM or a OSMR agonist with a type I IFN.

The present invention further relates to a recombinant expression vector comprising a nucleic acid encoding OSM or an OSMR agonist, and optionally a nucleic acid encoding type I interferon, operably linked to one or more control sequences that direct the production of the OSM or OSMR agonist, and optionally type I IFN in a suitable expression host. Host cells transfected or transformed with the expression vector mentioned herein above are also provided. Both vectors and transfected or transformed host cells may be used in gene therapy, in the manufacture of a medicament for enhancing the immunostimulatory activity of epithelial cells in a human.

The present invention also relates to an *in vitro* method for the preparation of stimulated lymphocytes CD8+ for use in adoptive immunotherapy, said method comprising:
a) treating human-derived epithelial cells or tissue with an effective dose of OSM, a OSMR agonist, or a combination of OSM or a OSMR agonist with a type I IFN, or of the recombinant expression vector provided by this invention;
b) irradiating the treated cells or tissue obtained from step a);
c) co-culturing the irradiated cells or tissue obtained from step b) with
   - peripheral blood mononuclear cells (PBMCs), or
   - enriched CD8+ lymphocytes and optionally antigen-presenting cells; and
d) optionally co-culturing the stimulated CD8+ lymphocytes obtained from step c) with treated and irradiated cells or tissue obtained from step b);
wherein the PBMCs, CD8+ lymphocytes, and epithelial cells or tissue are derived from the same human subject. Stimulated lymphocytes CD8+ obtainable by an *in vitro* method, methods of treatment, and uses of these stimulated lymphocytes CD8+ as a medicament and in the manufacture of a medicament for the treatment of cancer or infectious diseases are also described.

### Description of the drawings

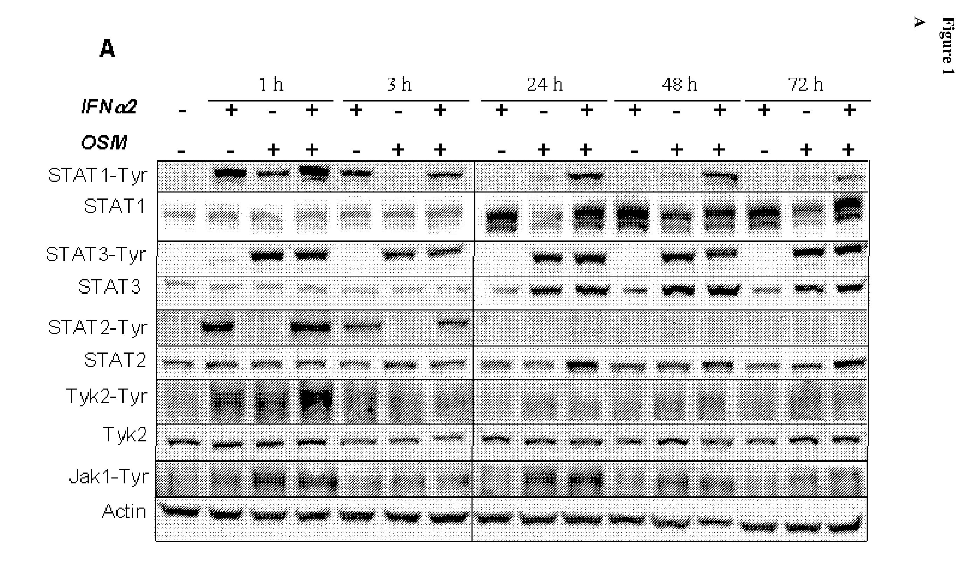

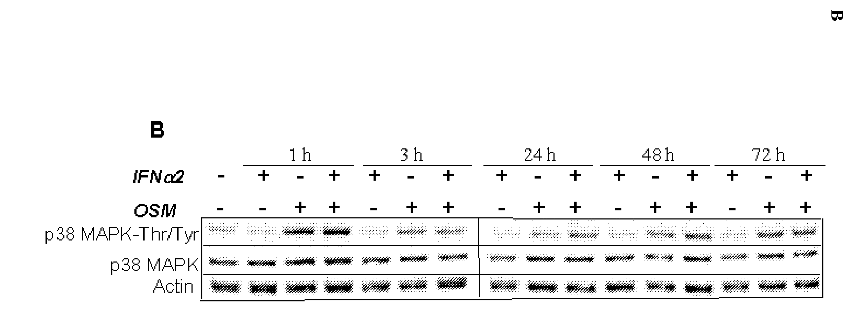

Figure 1. Jak/STAT signaling pathway and p38 MAPK activation in Huh7 cells treated with IFNα2, OSM, or the combination of the two, for the indicated periods of time. (A) Representative Western blot analysis of phosphorylated and total protein levels of STAT1, STAT2, STAT3, Tyk2 and Jak1 in Huh7 cells cultured in medium alone or in the presence of IFNα2 or OSM or IFNα2 plus OSM during 1, 3, 24, 48 and 72 h. (B) Representative Western blot analysis of phosphorylated and total protein of p38 MAPK levels in Huh7 cells untreated or treated with IFNα2 or OSM or IFNα2 plus OSM during 1, 3, 24, 48, and 72 h. Actin levels are shown as loading control.

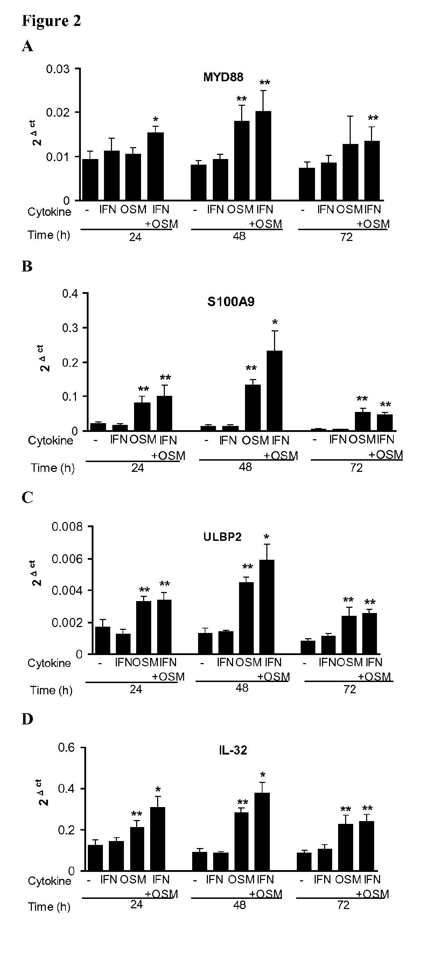

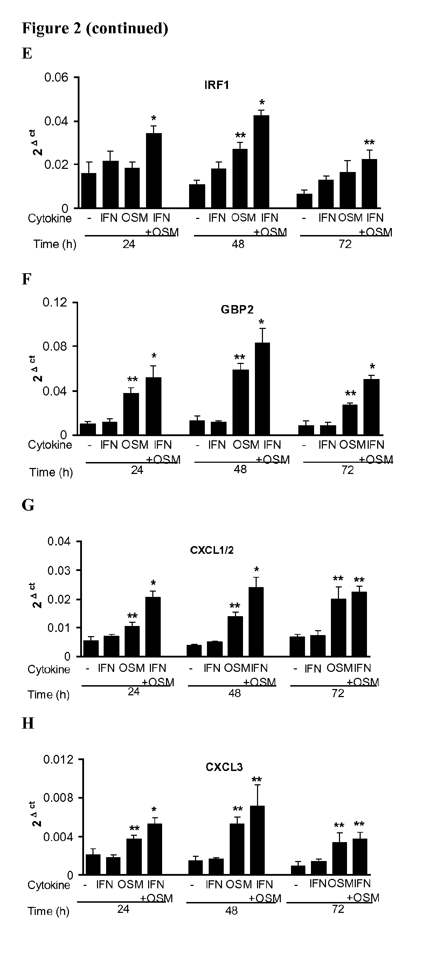

Figure 2. OSM upregulates genes that participate in the natural defense against infection. The transcriptional expression of MYD88 (a TLR adapter molecule) (A), S100A9 (a modulator of TLR function) (B), ULBP2 (a ligand for NKG2D that activates NK cells) (C), IL-32 (a proinflammatory molecule that activates macrophages) (D), IRF1 (E), GBP2 (F) and chemokine genes CXCL1/2 and CXCL3 (G and H) was determined by quantitative RT-PCR in Huh7 cells treated for the indicated periods of time with IFNα2, OSM, or the combination of the two cytokines or left untreated. Values are means ± SD of three experiments performed in quintuplicate * p<0.05 vs untreated, IFNα2 and OSM. ** p<0.05 vs untreated and IFNα2.

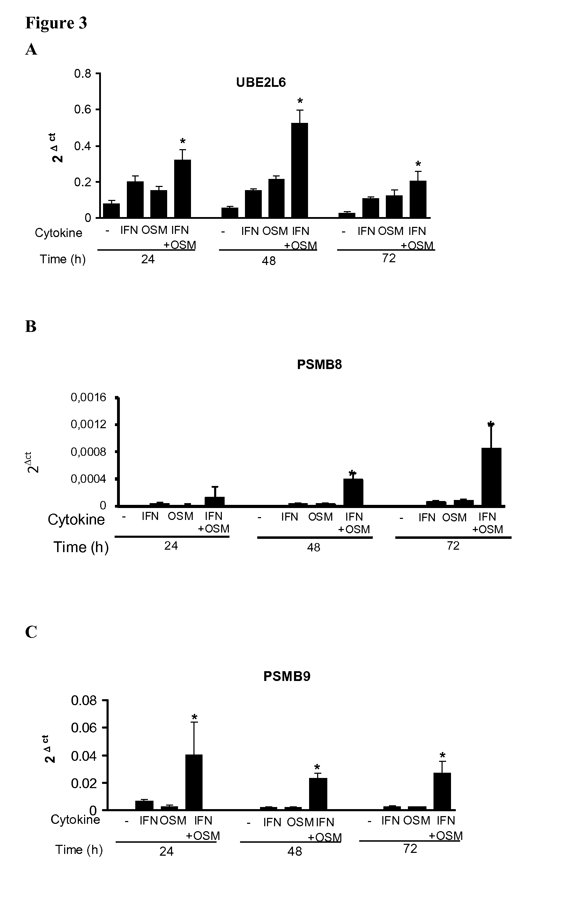

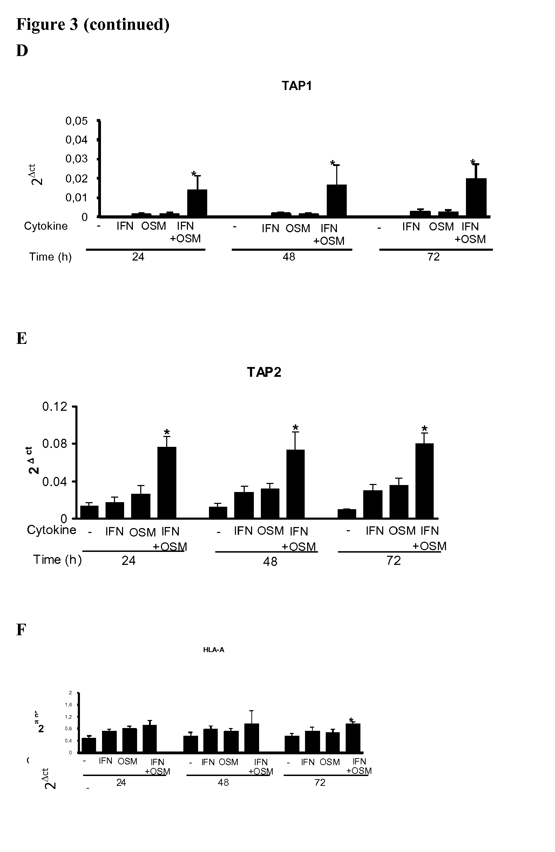

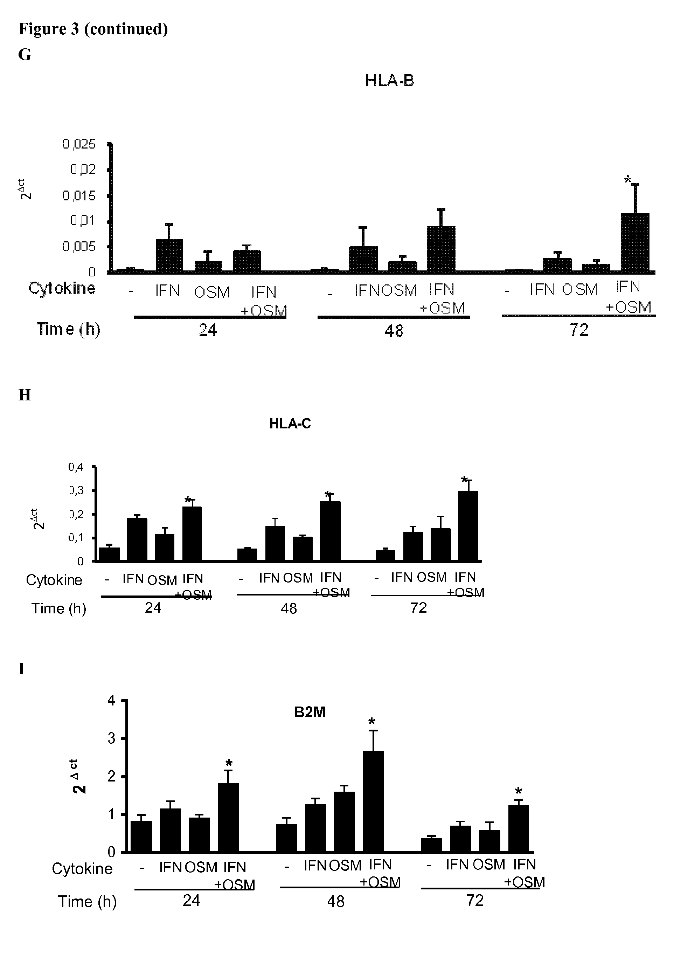

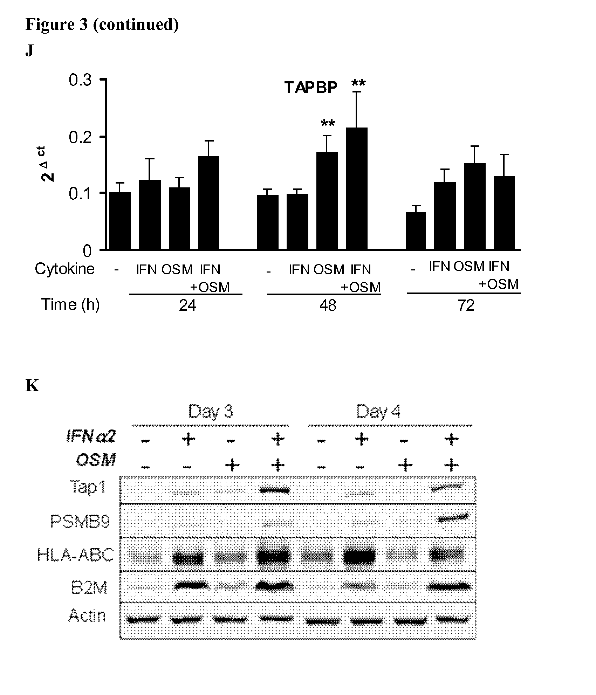

Figure 3. OSM synergizes with IFNα in the induction of genes involved in antigen processing and presentation in Huh7 cells. Determination by quantitative RT-PCR of mRNA levels of ubiquitin-conjugating enzyme UBE2L6 (A), immunoproteasome subunits PSMB8 (B) and PSMB9 (C), transporters associated with antigen processing TAP1 (D) and TAP2 (E), HLA class I A, B, and C (F, G, H), the TAP binding protein TAPBP (I), and β2 microglobulin (B2M) (J) in Huh7 cells treated for the indicated periods of time with IFNα2, OSM, or both or left untreated. Values are means ± SD of three experiments performed in quintuplicate. Western blot for TAP1, PSMB9, HLA-ABC and B2M in Huh7 cells treated for 3 and 4 days with IFNα2, OSM, or the combination of the two cytokines or left untreated. Actin levels are shown as loading control (K).* p<0.05 vs untreated, IFNα2 and OSM. ** p<0.05 vs untreated and IFNα2. Values are means ± SD of three experiments performed in quintuplicate.

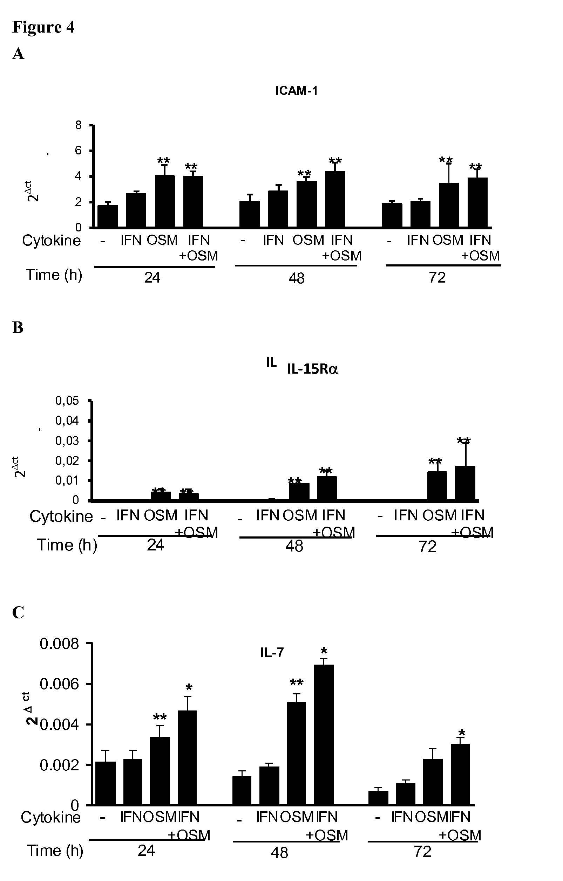

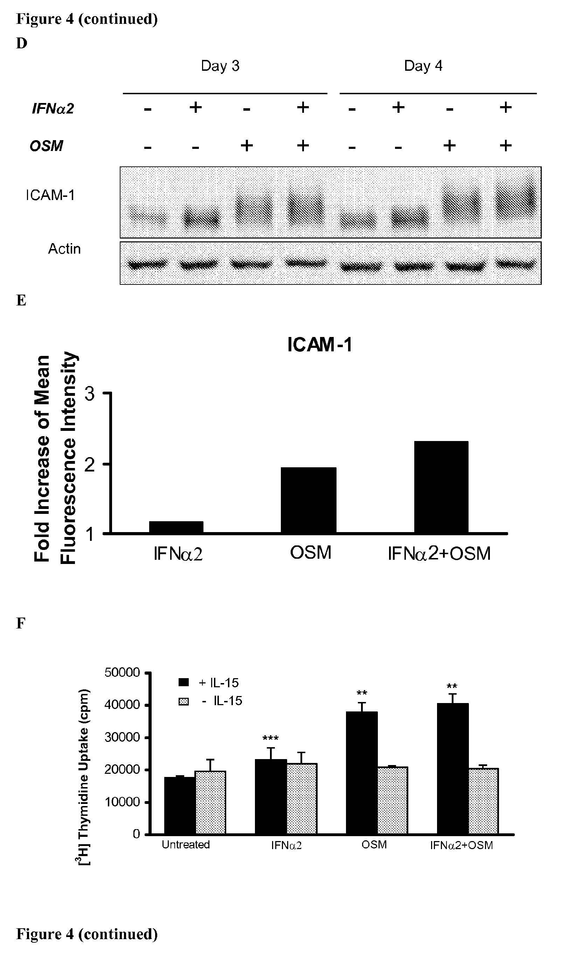

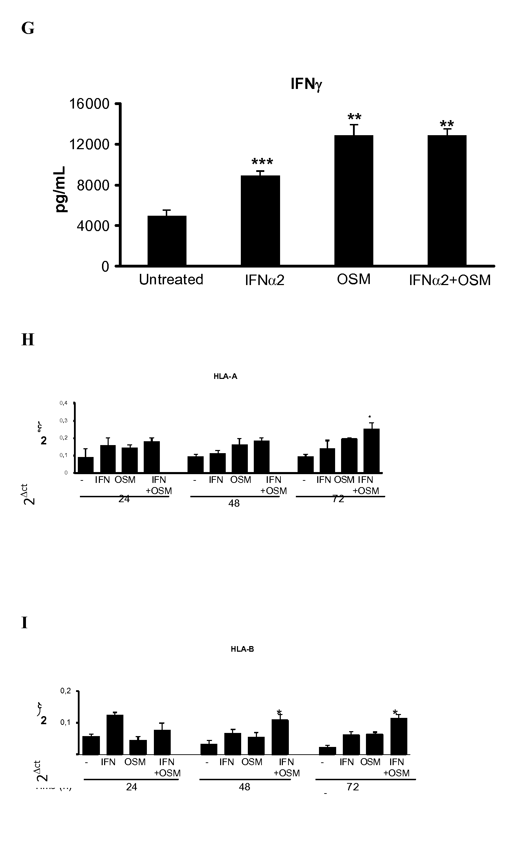

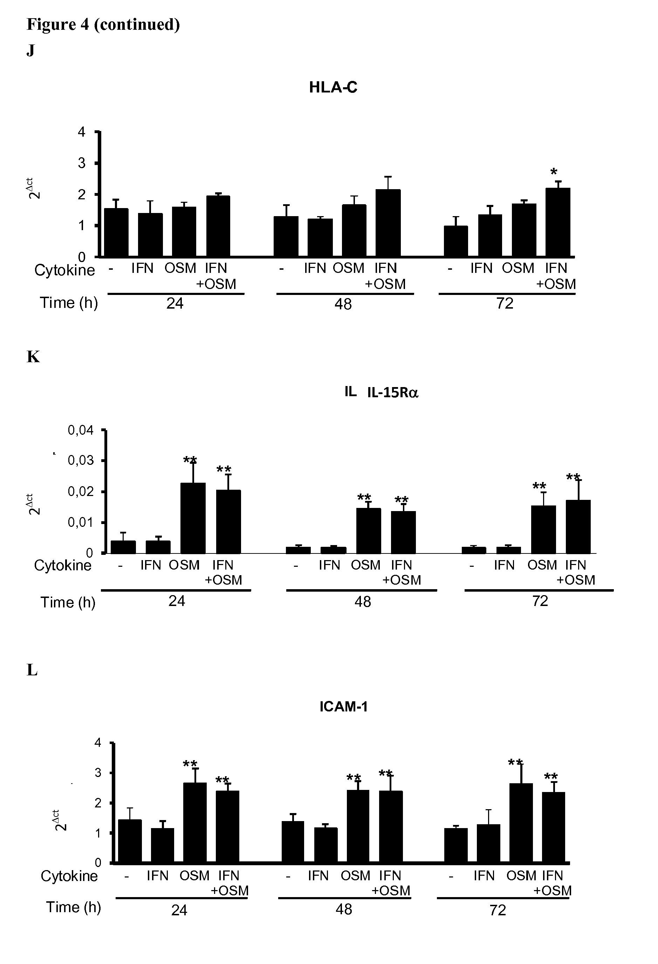

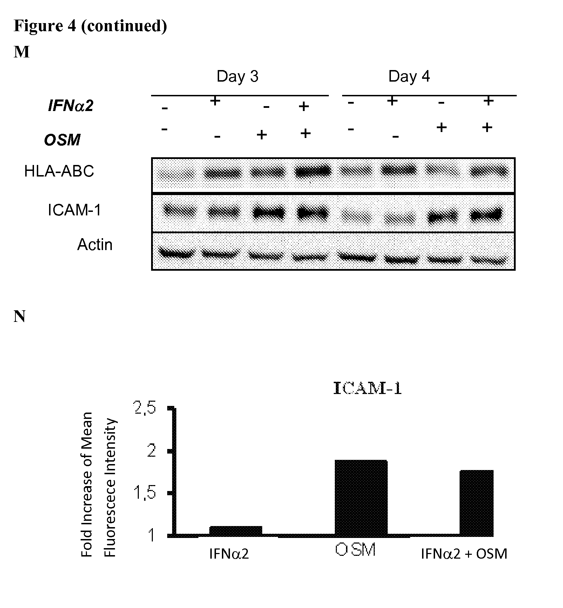

Figure 4. OSM upregulates genes that stimulate adaptive immunity and enhances IL-15 transpresentation and the immunostimulatory activity of liver epithelial cells. Transcriptional expression of ICAM-1 (A), IL-15Rα (B) and IL-7 (C) in Huh7 cells treated with IFNα2, OSM, or both or left untreated. Western blot for ICAM-1 in Huh7 cells treated with IFNα2, OSM, or both or left untreated (D). Flow cytometry studies to determine ICAM-1 (E) on the surface of Huh7 cells treated with IFNα2, OSM, or the combination of the two cytokines for four days. Values represent fold times versus non-treated cells .Proliferative activity of CTLL-2 cells (a CD8+ T cell line with IL-15 growth dependence) incubated with Huh7 cells treated for 3 days with IFNα2, OSM, or both or left untreated (F). Production of IFNγ by CTLs sensitized to the influenza virus peptide GILGFVFTL (SEQ ID NO: 46) incubated in presence of HepG2 cells presenting the peptide. HepG2 cells had been previously treated for 4 days with IFNα2, OSM, or both or left untreated (G). Determination by quantitative RT-PCR of mRNA levels of HLA-A, HLA-B, HLA-C, IL-15Rα and ICAM-1 (H-L) and Western blot for ICAM-1 and HLA-ABC (M) in HepG2 cells treated for the indicated periods of time with IFNα2, OSM, or the combination of the two cytokines or left untreated. Flow cytometry studies to determine ICAM-1 (N) on the surface of HepG2 cells treated with IFNα2, OSM, or the combination of the two cytokines for four days. Values are means ± SD of three experiments performed in quintuplicate or sextuplicate. * p<0.05 vs untreated, IFNα2 and OSM. ** p<0.05 vs untreated and IFNα2. *** p<0.05 vs untreated.

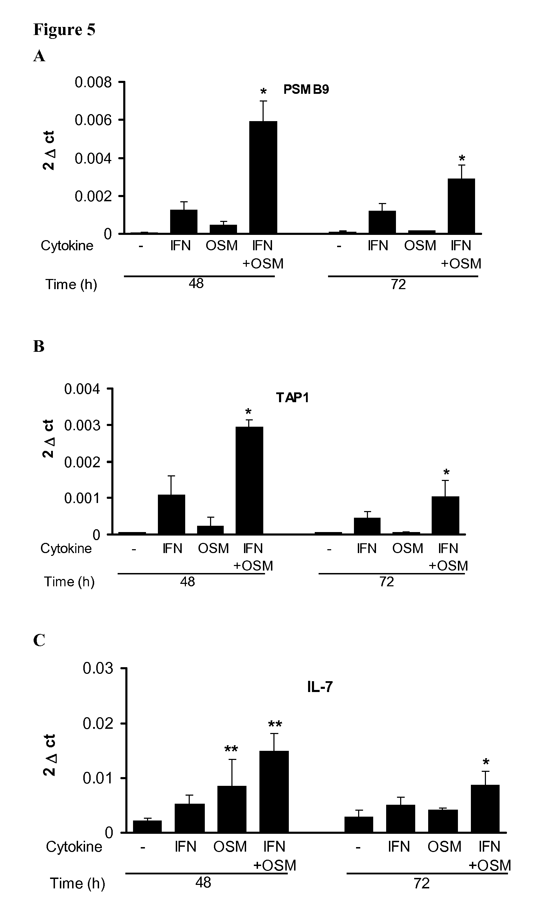

Figure 5. OSM enhances IFNβ-induced upregulation of genes involved in antigen processing and presentation (PSMB9, TAP1), and genes implicated in activation of adaptive immunity (IL-7). Determination of mRNA abundance was performed by quantitative RT-PCR in Huh7 cells treated for the indicated periods of time with IFNβ, OSM, or the combination of the two cytokines or left untreated. Values are means ± SD of two experiment performed in sextuplicate. * p<0.05 vs untreated, IFN and OSM. ** p<0.05 vs untreated and IFNβ.

### Disclosure of the invention

The present invention relates to the use of oncostatin M (OSM), a OSM receptor (OSMR) agonist, or a combination of OSM or a OSMR agonist with a type I interferon (type I IFN); in the manufacture of a medicament for enhancing the immunostimulatory activity of epithelial cells in a human. Similarly, the present invention relates to OSM, a OSMR agonist, or a combination of OSM or a OSMR agonist with a type I IFN for use in enhancing the immunostimulatory activity of epithelial cells in a human. There is also provided a method of enhancing the immunostimulatory activity of epithelial cells in a human, comprising administering to said human an effective amount of OSM, a OSMR agonist, or a combination of OSM or a OSMR agonist with a type I IFN. The term "OSM alone or combined with a type I IFN" is used herein to encompass oncostatin M (OSM), a OSM receptor (OSMR) agonist, or a combination of OSM with a type I interferon, or a combination of a OSMR agonist with a type I interferon.

The term "oncostatin M" or "OSM" as used herein refers to a cytokine originally isolated from medium conditioned by PMA-treated U-937 human histiocytic leukemia cells based on its ability to inhibit growth of A375 melanoma cells. The human OSM cDNA encodes a 252 amino acid pre-pro-OSM polypeptide with a 25 residue hydrophobic signal peptide and a hydrophilic C-terminal domain that are proteolytically processed to generate the 196 residue mature form of OSM. The listed accession number for this protein can be found in the publicly available protein database at http://www.expasy.org/uniprot/P13725. The amino acid sequence of human OSM is the following represented by SEQ ID NO. 1:

MGVLLTQRTLLSLVLALLFPSMASMAAIGSCSKEYRVLLGQLQKQTDLMQDTS
RLLDPYIRIQGLDVPKLREHCRERPGAFPSEETLRGLGRRGFLQTLNATLGCVLH
RLADLEQRLPKAQDLERSGLNIEDLEKLQMARPNILGLRNNIYCMAQLLDNSDT
AEPTKAGRGASQPPTPTPASDAFQRKLEGCRFLHGYHRFMHSVGRVFSKWGES
PNRSRRHSPHQALRKGVRRTRPSRKGKRLMTRGQLPR

Typical suitable OSMs include, but are not limited to human OSM of natural origin or recombinant human OSM, available for example from R&D Systems.

According to the "International Union of Pharmacology Committee on Receptor Nomenclature and Drug Classification", an agonist is a ligand that binds to a receptor and alters the receptor state resulting in a biological response. Conventional agonists increase receptor activity, whereas inverse agonists reduce it. As such, an inverse agonist is defined as a ligand that by binding to receptors reduces the fraction of them in an active conformation. Inverse agonists show a preferential binding affinity for the inactive conformation of the receptor. In systems where constitutive activity of the receptor can be seen, inverse agonists induce the opposite action of that of agonists.

The term "OSMR agonist" refers to a conventional agonist, i.e. a ligand that binds to the OSM receptor increasing its activity. An example of an OSMR agonist is provided in patent publication JP 2004026768 (Kanagawa Kagaku Gijutsu Akad).

The term "ligand" means any molecule capable of specifically binding to the OSMR. It includes peptides, polypeptides, membrane-associated or membrane-bound molecules, or complexes thereof, as well as small molecules.

"Small molecules" is defined as a molecule with a molecular weight that is less than 10 kD, typically less than 2 kD, and preferably less than 1 kD. Small molecules include, but are not limited to, inorganic molecules, organic molecules, organic molecules containing an inorganic component, molecules comprising a radioactive atom, synthetic molecules, peptide mimetics, and antibody mimetics.

The term "interferon type I," "IFN type I," or equivalent denominations, refers to the proteins interferon-alpha (IFN- α) and interferon-beta (IFN-β), and the family of highly homologous species-specific proteins that inhibit viral replication and cellular proliferation and modulate immune response.

The term "interferon-alpha" as used herein encompasses IFN-α of natural origin as well as recombinant proteins. Examples of IFN-α include interferon alpha-2b such as Intron-A interferon available from Schering Corporation; recombinant interferon alpha-2a such as Roferon interferon available from Hoffmann-La Roche; recombinant interferon alpha-2c such as Berofor alpha 2 interferon available from Boehringer Ingelheim; interferon alpha-nl, a purified blend of natural alpha interferons such as Sumiferon available from Sumitomo or as Wellferon interferon alpha-n1 (INS) available from Glaxo-Wellcome; or a consensus interferon alpha such as those described in Patent Nos. US4897471 and US4695623 (especially Examples 7, 8 or 9 thereof), the specific product available from Amgen; interferon alpha-5; or interferon alpha-n3, a mixture of natural interferon alphas made by Interferon Sciences and available from the Purdue Frederick Co. under the Alferon tradename. The use of interferon alpha-2a or alpha-2b is preferred. Since interferon alpha 2b, among all interferons, has the broadest approval throughout the world, it is most preferred. The manufacture of interferon alpha 2b is described in Patent No. US4530901.

The term "interferon-beta" is meant to encompass IFN-β of natural origin as well as recombinant proteins. Examples of IFN-β include, without being limited to, interferon-beta-1a and interferon-beta-1b. Interferon-beta-1a is sold by Biogen under the trademark Avonex, and by Serono under the trademark Rebif. The other form, interferon-beta-1b, is sold under the trademark Betaseron by Berlex. The IFN-beta-1a in Avonex® is the glycosylated, native human sequence whereas the IFN-beta-1b in Betaseron® is the unglycosylated, serine 17-substituted, native human sequence.

OSM and IFN type I proteins may be obtained from a variety of cell sources that synthesize bioactive OSM or IFN type I proteins including, for example, cells that naturally produce these proteins, or cells transfected or transformed with recombinant DNA molecules capable of directing the synthesis or secretion of the proteins. Alternatively, OSM and IFN type I proteins may be synthesized by chemical synthetic methods, including but not limited to, solid phase peptide synthesis.

The term "recombinant protein," as used in the present invention in relation to OSM and IFN type I, refers to these proteins as obtained by DNA recombinant techniques from prokaryotic or eukaryotic host cells as well as its salts, functional derivatives, variants, analogs, and fragments.

Typically, recombinant OSM, recombinant IFN-α, and recombinant IFN-beta-1b may be obtained in a genetically engineered *Escherichia coli* bacterium comprising DNA that codes for the human protein, followed by fermentation in a suitable nutrient medium. The IFN-beta-1a in Avonex® is produced in Chinese Hamster ovary cells using recombinant DNA technology.

The term "salts" herein refers to both salts of carboxyl groups and to acid addition salts of amino groups of the OSM, OSMR agonists, and IFN type I molecules or analogs thereof Salts of a carboxyl group may be formed by means known in the art and include inorganic salts, for example, sodium, calcium, ammonium, ferric, or zinc salts, and the like, and salts with organic bases as those formed, for example, with amines, such as triethanolamine, arginine, lysine, piperidine, procaine, and the like. Acid addition salts include, for example, salts with mineral acids, such as, for example, hydrochloric acid or sulfuric acid, and salts with organic acids, such as, for example, acetic acid or oxalic acid. Of course, any such salt must retain the biological activity of OSM or IFN type I. For therapeutic use, salts of OSM and IFN type I are those wherein the counter-ion is pharmaceutically acceptable. Non-pharmaceutically acceptable salts are also encompassed in the ambit of the present invention since they can be used in the production of pharmaceutically acceptable end products.

"Functional derivatives" as used herein covers derivatives which may be prepared from the functional groups which occur as side chains on the residues or the N- or C-terminal groups, by means known in the art, and are included in the invention as long as they remain pharmaceutically acceptable, i.e., they do not destroy the biological activity of the proteins as described above, i.e., the ability to bind the corresponding receptor and initiate receptor signaling, and do not confer toxic properties on compositions containing it. Derivatives may have chemical moieties, such as carbohydrate or phosphate residues, provided such a derivative retains the biological activity of the protein and remains pharmaceutically acceptable.

For example, derivatives may include aliphatic esters of the carboxyl groups, amides of the carboxyl groups by reaction with ammonia or with primary or secondary amines, N-acyl derivatives or free amino groups of the amino acid residues formed with acyl moieties (e.g., alkanoyl or carbocyclic aroyl groups), or O-acyl derivatives of free hydroxyl group (e.g., that of seryl or threonyl residues) formed with acyl moieties. Such derivatives may also include for example, polyethylene glycol side-chains, which may mask antigenic sites and extend the residence of the molecule in body fluids.

Of particular importance is a protein that has been derivatized or combined with a complexing agent to be long lasting. For example, pegylated versions, or proteins genetically engineered to exhibit long lasting activity in the body, can be used according to the present invention.

A "variant" according to the present invention refers to a molecule, which is substantially similar to either the entire proteins defined above or a fragment thereof. Variant peptides may be conveniently prepared by direct chemical synthesis of the variant peptide, using methods well known in the art. Of course, such variant would have similar receptor binding and signal initiating activity as the corresponding naturally occurring protein.

Variations in the protein primary structure, as well as variations in higher levels of structural organization, e.g. variation in the type of covalent bonds linking the amino acid residues or addition of groups to the terminal residues of the protein, are within the scope of the invention. Further, the protein molecules may include conservative or non-conservative alterations in the amino acid sequence that result in silent changes that preserve the functionality of the molecule including, for example, deletions, additions, and substitutions. Such altered molecules may be desirable where they provide certain advantages in their use. As used herein, conservative substitutions would involve the substitution of one or more amino acids within the sequence of the corresponding protein with another amino acid having similar polarity and hydrophobicity/hydrophilicity characteristics resulting in a functionally equivalent molecule. Such conservative substitutions include but are not limited to substitutions within the following groups of amino acids: glycine, alanine; valine, isoleucine, leucine; aspartic acid, glutamic acid; asparagine, glutamine; serine, threonine; lysine, arginine; phenylalanine, tyrosine; and methionine, norleucine.

Amino acid sequence variants of the proteins previously defined can be prepared by mutations in the DNAs, which encode the synthesized derivatives. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure.

At the genetic level, these variants ordinarily are prepared by site-directed mutagenesis of nucleotides in the DNA encoding the peptide molecule, thereby producing DNA encoding the variant, and thereafter expressing the DNA in recombinant cell culture. The variants typically exhibit the same qualitative biological activity as the non-variant peptide.

An "analog" of the proteins defmed above, according to the present invention, refers to a non-natural molecule, which is substantially similar to either, the entire molecules or to an active fragment thereof. Such analog would exhibit the same activity as the corresponding naturally occurring protein.

A "fragment" according to the present invention refers to any subset of the molecules, that is, a shorter peptide, which retains the desired biological activity. Fragments may readily be prepared by removing amino acids from either end of the molecule and testing the resultant for its properties as a receptor agonist. Proteases for removing one amino acid at a time from either the N-terminal or the C-terminal of a polypeptide are known in the art.

The term "immunostimulatory activity of epithelial cells" refers to the development of a cellular immune response effected by the epithelial cells.

A "cellular immune response" refers to an immune response mediated by T-lymphocytes, by other white blood cells, or a combination of the two. One important aspect of cellular immunity involves an antigen-specific response by cytotoxic T-cells (CTL). CTLs have specificity for peptide antigens that are presented in association with proteins encoded by the major histocompatibility complex (MHC) and expressed on the surfaces of cells. CTLs help induce and promote the intracellular destruction of intracellular pathogens, or the lysis of cells infected with such pathogens. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines, and other such molecules produced by activated T-cells and other white blood cells, including those derived from CD4+ and CD8+ T-cells.

OSM alone or combined with an IFN type I confers immunogenic properties to the epithelial cells since it stimulates the process of antigen processing and presentation in said cells.

The term "immunogenicity" refers to the ability of a particular substance, which is called the antigen, to provoke an immune response (see, e.g., Roitt: Essential Immunology (11th Edition, Blackwell) for further definition of immunogenicity). When used in connection with OSM alone or combined with an IFN type I, it indicates the ability of these proteins to induce a response from the immune system, by activating the antigen processing and presentation machinery in epithelial cells. OSM alone or combined with an IFN type I may also be considered as a substance that increases the immunogenicity of epithelial cells, since it activates antigen production and is displayed at the cellular membranes, where the antigen ultimately provokes an immune response.

The process of antigen processing and presentation in epithelial cells involves the generation of antigens within said cells, i.e. the fragmentation (proteolysis) of proteins expressed by pathogens that have infected the epithelial cell, as well as aberrant proteins encoded by mutant genes, like mutated genes in cancer cells. These endogenous antigens that are degraded into fragments (e.g., peptides) within the cell, are then associated with MHC molecules, and finally displayed or expressed-presented-at the surface of the cell nestled within a class I histocompatibility molecule, where they can be recognized by the T cell receptor on a T cell, particularly CD8+ T cells, which have the machinery to destroy the infected or tumoral cell.

As such, OSM alone or combined with an IFN type I increases the antigenicity of infected or tumoral epithelial cells, making these cells process and present more antigens at their surface, and therefore becoming more visible and reactive to immune responses, resulting finally into a selective destruction of the same cells.

Therefore, OSM alone or combined with an IFN type I represent useful agents in the immunotherapy of cancer and diseases caused by infectious pathogens, like virus, bacteria, fungi, and parasites, since said proteins can selectively activate the process of destroying only those epithelial cells that are infected with pathogens or are tumoral.

The term "immunotherapy" as used herein refers to a therapeutic or prophylactic treatment based on the activation of a cellular immune response.

In one embodiment of the present invention, the cellular immune response may be an innate immune response or an adaptive immune response. Preferably, the cellular immune response is an adaptive immune response.

In one embodiment of the present invention, the immunostimulatory activity of epithelial cells comprises the proliferation of T lymphocytes, the transpresentation of interleukin 15 (IL-15) to NK or CD8+ cells, or the production of interferon gamma (IFN-γ).

A further embodiment of the present invention relates to the use of OSM, a OSMR agonist, or a combination of OSM or a OSMR agonist with an interferon type I, in the manufacture of a medicament for enhancing the immunostimulatory activity of epithelial cells in a human, wherein the epithelial cells are infected with a cellular pathogen or are tumor epithelial cells.

The cellular pathogen infecting the epithelial cell is meant to include pathogenic virus, bacteria, fungi, and parasites, particularly retroviruses, adenovirus, papillomavirus, herpes viruses, cytomegalovirus, adeno-associated viruses, hepatitis viruses, pneumoviruses, noroviruses, rotaviruses, astroviruses, paramyxoviruses, mumps virus, viral proteins, viroids, and the like; meningococcus, pneumococcus, mycobacteria, *Salmonella* sp., *Shigella* sp., *Staphylococcus* sp., *Campylobacter jejuni*, *Clostridium* sp., *Escherichia coli*, *Yersinia* sp., *Bordetella pertussis*, *Treponema* sp., and the like; parasites such as *Leishmania* sp., *Toxoplasma* sp., *Schistosoma* sp., *Clonorchis* sp., or *Plasmodium* sp.; or fungi like *Histoplasma* sp., *Aspergillus* sp., *Candida* sp., *Cryptococcus* sp., *Pneumocystis* sp., and the like.

The tumor epithelial cells may be selected from carcinomas such as adenocarcinoma (bronchiolo-alveolar adenocarcinoma, clear cell adenocarcinoma, follicular adenocarcinoma, mucinous adenocarcinoma, papillary adenocarcinoma, scirrhous adenocarcinoma, sebaceous adenocarcinoma, adrenocortical carcinoma, carcinoid tumor, acinar cell carcinoma, adenoid cystic carcinoma, ductal carcinoma, endometrioid carcinoma, pancreatic adenocarcinoma, gastric carcinoma, colorectal cancer, hepatocellular carcinoma, noninfiltrating intraductal carcinoma, islet cell carcinoma, lobular carcinoma, mucoepidermoid carcinoma, neuroendocrine carcinoma, renal cell carcinoma, signet ring cell carcinoma, skin appendage carcinoma, cholangiocarcinoma, choriocarcinoma, cystadenocarcinoma, Klatskin's tumor, extramammary Paget's disease); adenosquamous carcinoma; basal cell carcinoma; basosquamous carcinoma; Ehrlich tumor carcinoma; giant cell carcinoma; carcinoma in situ (cervical intraepithelial neoplasia and prostatic intraepithelial neoplasia); Krebs 2 carcinoma; large cell carcinoma; Lewis lung carcinoma; non-small-cell lung carcinoma; papillary carcinoma; squamous cell carcinoma (Bowen's disease); transitional cell carcinoma; verrucous carcinoma.

The tumor epithelial cells may also be selected from adnexal and skin appendage neoplasms such as sebaceous adenocarcinomas, skin appendage carcinoma; basal cell neoplasms such as basal cell carcinomas (basal cell nevus syndrome), basosquamous carcinoma, and pilomatrixoma; cystic, mucinous, and serous neoplasms such as mucinous adenocarcinomas, mucoepidermoid carcinoma, signet ring cell carcinoma (Krukenberg tumor), cystadenocarcinoma (mucinous cystadenocarcinoma, papillary cystadenocarcinoma, serous cystadenocarcinoma), cystadenoma (mucinous cystadenoma, papillary cystadenoma, serous cystadenoma), mucoepidermoid tumor, and pseudomyxoma peritonei; ductal, lobular, and medullary neoplasms such as ductal carcinoma (ductal breast carcinoma, pancreatic ductal carcinoma), intraductal noninfiltrating carcinoma (mammary Paget's disease), lobular carcinoma, medullary carcinoma, extramammary Paget's disease, intraductal papilloma; fibroepithelial neoplasms such as adenofibroma, brenner tumor; mesothelial neoplasms such as adenomatoid tumor, mesothelioma (cystic mesothelioma); and squamous cell neoplasms such as acanthoma, papillary carcinoma, squamous cell carcinoma (Bowen's disease), verrucous carcinoma, papilloma (inverted papilloma).

OSM, a OSMR agonist, or a combination of OSM or a OSMR agonist with an interferon type I may be administered together as separate pharmaceutical formulations or as part of the same unitary dosage form. Alternatively, OSM, a OSMR agonist, or a combination of OSM or a OSMR agonist with an interferon type I may be administered separately but as part of the same therapeutic regimen. The two components, if administered separately, need not necessarily be administered at essentially the same time, although they can if so desired. Thus, OSM, a OSMR agonist, or a combination of OSM or a OSMR agonist with an interferon type I may be administered as separate dosages or dosage forms, but at the same time. Optionally, the separate administration of OSM, a OSMR agonist, or a combination of OSM or a OSMR agonist with an interferon type I may be performed at different times and in any order.

In one embodiment of the present invention, there is provided a product comprising OSM or a OSMR agonist, and an IFN type I as a combined preparation for simultaneous, separate, or sequential use for enhancing the immunostimulatory activity of epithelial cells in a human. In a further embodiment, there is provided a product containing OSM or a OSMR agonist, and an IFN type I as a combined preparation for simultaneous, separate, or sequential use for enhancing the immunostimulatory activity of epithelial cells in a human.

As such, the present invention further relates to combining separate pharmaceutical formulations in kit form. For example, a kit may comprise two separate pharmaceutical formulations comprising: 1) OSM or a OSMR agonist; and 2) an IFN type I. The kit also comprises a container for the separate formulations, such as a divided bottle or a divided foil packet. Additional examples of containers include syringes, boxes, bags, and the like. Typically, a kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

The product or kit of the present invention is particularly suited for enhancing the immunostimulatory activity of epithelial cells in a human. In one embodiment of the present invention, the product or kit comprising OSM, a OSMR agonist, or a combination of OSM or a OSMR agonist with an interferon type I is used in the immunotherapy of cancer and diseases caused by infectious pathogens, like virus, bacteria, fungi, and parasites.

In a further embodiment, the present invention concerns the above-mentioned uses of OSM alone or combined with IFN type I in the manufacture of a medicament for enhancing the immunostimulatory activity of epithelial cells in a human, wherein OSM alone or combined with IFN type I is formulated in a pharmaceutical composition comprising a therapeutically effective amount of OSM alone or combined with IFN type I, and a pharmaceutically acceptable carrier. A therapeutically effective amount in this context is an amount sufficient to enhance the immunostimulatory activity of epithelial cells in a human, in a clinically significant manner.

OSM alone or combined with a IFN type I may be formulated into various pharmaceutical forms for administration purposes. As appropriate compositions there may be cited all compositions usually employed for systemically administering drugs. To prepare the pharmaceutical compositions of this invention, an effective amount of OSM alone or combined with a IFN type I as the active ingredient is combined in intimate admixture with a pharmaceutically acceptable carrier, which carrier may take a wide variety of forms depending on the manner of preparation desired for administration. These pharmaceutical compositions are desirable in unitary dosage form suitable, particularly, for administration orally, rectally, transdermally, by inhalation, or by parenteral routes (i.e., subcutaneous, intravenous, intramuscular, or intraperitoneal). OSM alone or combined with an IFN type I can be administered by the oral route in solid dosage forms, such as tablets, capsules, and powders, or in liquid dosage forms, such as elixirs, syrups, and suspensions. The pharmaceutical compositions of this invention can also be administered parenterally, in sterile liquid dosage forms. Preferred routes of administration are those parenteral routes, particularly by intramuscular, intravenous, or subcutaneous injections.

Formulations for parenteral administration may be in the form of aqueous or nonaqueous isotonic sterile injection solutions or suspensions. Injectable solutions, for example, may be prepared, in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration-enhancing agent and/or a suitable wetting agent, optionally combined with suitable additives of any nature in minor proportions, which additives do not introduce a significant deleterious effect on the skin. These latter suitable additives may be antioxidants, preservatives, stabilizing agents, emulsifiers, salts for influencing the osmotic pressure, and/or buffer substances.

Alternatively, OSM alone or combined with an IFN type I may be may be delivered to the epithelial cells formulated in liposomes. Liposomes can be prepared using conventional techniques including sonication, chelate dialysis, homogenization, solvent infusion coupled with extrusion, freeze-thaw extrusion, microemulsification, as well as others. Reagents used to crosslink a liposome or other lipid-containing agent to the proteins of the present invention comprise a phospholipid derivative to anchor one end of the crosslink in the lipid layer and a reactive group at the other end to provide a point of attachment to the target biomolecule. Polymerized liposomes or liposomes coated with polymer may as well be used. Such polymers may stabilize the liposome, reduce its clearance from the body, and/or reduce its immunogenicity. The liposome may be loaded with a functional moiety such as a diagnostic or therapeutic agent either during or after its formation. The agent may be contained in an aqueous core of the liposome or can be incorporated into or attached to its surrounding membrane.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The daily dosage of the compounds according to the invention shall vary with the mode of administration, the treatment desired, and the severity of the disorder.

In general it is contemplated that an effective daily amount of OSM may range from about 0.05 to about 5.0 mg per patient. In the case of an IFN type I, the effective daily amount would be from 1 to 1,000 µg / mL. IFNα2a is usually administered at 11.1µg/0.5mL, 22.2µg/0.5mL, 33.3µg/0.5mL, daily or three times a week. IFNα2b is usually administered at 0.038mg/1mL, 0.069mg/1mL, 0.087mg/1.5mL, 0.144mg/1.5mL, 0.192mg/1mL, 0.288mg/1.5mL, by injection three or five times a week. IFNβ1a is usually administered at 8.8 µg/0.5mL, 22µg/0.5mL, or 44µg/0.5mL injected subcutaneously three times per week. IFNβ1b is usually administered at 0.0625mg / 0.25mL and increased to 0.25mg/1mL injected subcutaneously every other day.

It may be appropriate to administer the required dose as one, two, three, four or more sub-doses at appropriate intervals throughout the day. Furthermore, it is evident that said effective daily amount may be lowered or increased depending on the response of the treated subject and/or depending on the evaluation of the physician prescribing the compounds of the instant invention. The effective daily amount ranges mentioned hereinabove are therefore only guidelines.

OSM alone or combined with an IFN type I may be provided as polypeptides, for example as recombinant proteins or, alternatively, one or both of said components may be provided as a polynucleotide comprising a nucleotide sequence encoding said components.

In a preferred embodiment, the polynucleotide encoding said components is comprised into a vector. In one embodiment of the present invention, there is provided a recombinant expression vector comprising a nucleic acid encoding OSM or a OSMR agonist, and optionally a nucleic acid encoding an interferon type I, operably linked to one or more control sequences that direct the production of the OSM or OSMR agonist, and optionally the IFN type I in a suitable expression host.

In some cases, the nucleic acid encoding the different polypeptide chains of OSM or a OSMR agonist, and of IFN type I can be provided in the same expression vector.

As a person skilled in the art will understand, instead of the combined administration of a polynucleotide comprising a nucleotide sequence encoding OSM or a OSMR agonist and a polynucleotide comprising a nucleotide sequence encoding an IFN type I for enhancing the immunostimulatory activity of epithelial cells in a human, another way of implementing the present invention consists of administering a recombinant expression vector comprising both polynucleotides, i.e. a vector comprising the nucleotide sequence encoding OSM or a OSMR agonist and the nucleotide sequence encoding an IFN type I. When the vector is expressed in the receptor host, it will thus produce the corresponding proteins carrying out the aforementioned therapeutic effect.

Vectors suitable to accommodate the polynucleotides forming the compositions and kits of the invention include a plasmid or a vector that, when introduced into the host cell, it integrates or not into the genome of said cell. Said vector can be obtained by conventional methods known by persons skilled in the art and can be found in, for example, Sambrook, et al., 2001.

Nevertheless, in the scope of the present invention, the vector of the invention is preferably a suitable viral or non-viral vector for its use in gene therapy; by way of a non-limiting illustration, said vectors can be viral vectors based on retroviruses, adenoviruses, alphaviruses, etc., or in case of non-viral vectors, the vectors can be DNA-liposome, DNA-polymer, DNA-polymer-liposome complexes, etc. (Hung, 1999). Said viral and non-viral vectors comprising a nucleotide sequence encoding OSM alone or combined with a IFN type I can be directly administered to the human body by conventional methods.

In a particular embodiment, the vector is a viral vector, which in other particular embodiment is an alphavirus, such as Semliki Forest virus, Sindbis, and Venezuelan Equine encephalitis (VEE); a high capacity adenovirus; a conditionally replicative adenovirus; or an adenoasociated virus. In a preferably particular embodiment, the alphavirus is a Semliki Forest virus.

Said vectors can be alternatively used to transform, transfect, or infect cells, preferably mammalian cells, including human cells, for example, *ex vivo*, and, subsequently implant them into the human body to obtain the desired therapeutic effect. For their administration to the subject, said cells will be formulated in a suitable medium that does not adversely affect their viability. Likewise, as persons skilled in the art will understand, said vector may comprise, among others, multiple cloning sites, expression-regulating sequences, suitable replication origins for the host cell in which the vector is to be introduced, selection markers, etc.

The vector of the present invention comprise one or more control or expression-regulating sequences preceding the polynucleotide that are operatively linked to the said polynucleotide to direct the production of the OSM or OSMR agonist, and optionally a IFN type I in a suitable expression host. As used in this description, the expression "operatively linked" means that the polynucleotide encoding the OSM or OSMR agonist and the polynucleotide encoding the IFN type I are within the correct reading frame for their expression under the control of the said control or expression-regulating sequences.

The regulating sequences useful for the present invention can be nuclear promoting sequences or, alternatively, enhancer sequences and/or other regulating sequences increasing the expression of the heterologous nucleic acid sequence. The promoter can be constitutive or inducible. If the constant expression of the heterologous nucleic acid sequence is desired, then a constitutive promoter is used. Examples of well-known constitutive promoters include the cytomegalovirus (CMV) immediate-early promoter, the Rous sarcoma virus promoter, and the like. A number of other examples of constitutive promoters are well known in the art and can be used in implementing the invention. If the controlled expression of the heterologous nucleic acid sequence is desired, then an inducible promoter must be used. In a non-induced state, the inducible promoter must be "silent." "Silent" is understood to mean that in the absence of an inducer, little or no expression of the heterologous nucleic acid sequence is detected; in the presence of an inducer, however, the expression of the heterologous nucleic acid sequence occurs. The expression level can be frequently controlled by varying the concentration of the inducer. By controlling the expression, for example, by varying the concentration of the inducer such that an inducible promoter is stimulated more strongly or weakly, the concentration of the transcribed product of the heterologous nucleic acid sequence can be affected. In the event that the heterologous nucleic acid sequence encodes a gene, the synthesized amount of protein can be controlled. It is thus possible to vary the concentration of the therapeutic product. Examples of well-known inducible promoters are an androgen or estrogen-responsive promoter, a doxycicline-responsive promoter, a metallothionein promoter, or a promoter responding to ecdysone. Other various examples are well known in the art and can be used in implementing the invention. In addition to constitutive and inducible promoters (which usually work in a great variety of cell or tissue types), tissue-specific promoters can be used to reach the expression of the specific heterologous nucleic acid sequence in cells or tissues. Well known examples of tissue-specific promoters include liver specific promoters like the albumin, alpha-1 antitrypsin, or hemopexin promoters (Kramer, et al., 2003), several muscle-specific promoters including: skeletal α-actin promoter, cardiac actin promoter, skeletal troponin C promoter, slow contraction cardiac troponin C promoter and the creatine kinase promoter/enhancer, or tumor specific promoters like α-fetoprotein promoter for human hepatocellular carcinoma or prostate specific antigen promoter for prostate cancer. There are a number of muscle-specific promoters that are well known in the art and can be used in implementing the invention (for a review of muscle-specific promoters see Miller, et al., 1993).

"Heterologous" as used herein means "of different natural origin" or represents a non-natural state. For example, heterologous refers to a nucleotide sequence derived from and inserted into the same natural, original cell type, but which is present in a non-natural state, e.g. a different copy number, or under the control of different regulatory elements. Similarly, if a host cell is transformed with a DNA or gene derived from another organism, particularly from another species, that gene is heterologous with respect to that host cell and also with respect to descendants of the host cell which carry that gene.

The vectors of the present invention may be delivered to the host cells by transfection. Conventional transfection techniques include a variety of art-recognized techniques for introducing foreign nucleic acid (e.g., DNA or RNA) into a host cell, including calcium phosphate or calcium chloride co-precipitation, DEAE-dextran-mediated transfection, lipofection, (micro)injection, or electroporation.

A further embodiment of the present invention relates to a host cell comprising the recombinant expression vector as defined herein above.

A "host cell" refers to a prokaryotic or eukaryotic cell that contains heterologous DNA that has been introduced into the cell by any means, e.g., electroporation, calcium phosphate precipitation, microinjection, transformation, viral infection, and the like.

The recombinant expression vectors and host cells of the present invention are therefore useful in the manufacture of a medicament for enhancing the immunostimulatory activity of epithelial cells in a human. As such, they can be appropriately conditioned with one or more pharmaceutically acceptable excipients, thereby constituting a pharmaceutical formulation.

In a further embodiment, the present invention relates to an *in vitro* method for the preparation of stimulated lymphocytes CD8+ for use in adoptive immunotherapy, said method comprising:
a) treating human-derived epithelial cells or tissue with an effective dose of OSM, a OSMR agonist, or a combination of OSM or a OSMR agonist with IFN type I, or the recombinant expression vector provided by the present invention;
b) irradiating the treated cells or tissue obtained from step a);
c) co-culturing the irradiated cells or tissue obtained from step b) with
   ● peripheral blood mononuclear cells (PBMCs), or
   ● enriched CD8+ lymphocytes and optionally antigen-presenting cells; and
d) optionally, co-culturing the stimulated CD8+ lymphocytes obtained from step c) with treated and irradiated cells or tissue obtained from step b);
wherein the PBMCs, CD8+ lymphocytes, and epithelial cells or tissue are derived from the same human subject.

Methods of using CD8+ lymphocytes for adoptive cell therapy in treatment of human subjects are known to clinicians skilled in the art. CD8+ lymphocytes prepared according to the methods described herein and known in the art can be used in such methods. For example, adoptive cell therapy using anti-tumor cytotoxic T-cell lines, have been tested in the clinic in patients with solid neoplasia (Turin, et al., 2007).

The present invention provides methods for enhancing adoptive immunotherapy by providing a patient with a level of enhanced immunity by stimulating cells ex vivo, and then readministering them to the patient. The cells are histocompatible with the subject, and may be allogenic or autologous. They are preferably derived from the same patient to be treated.

The *in vitro* method of preparation of stimulated lymphocytes CD8+ comprises isolating peripheral blood mononuclear cells (PBMCs) from a patient; isolating epithelial cells from the same patient and treating them with an effective dose of OSM, a OSMR agonist, or a combination of OSM or a OSMR agonist with a IFN type I, or the recombinant expression vector of the present invention; irradiating the treated cells; and expanding the cells in culture to very high numbers in a culture media. These expanded cells that comprise stimulated lymphocytes CD8+ may then be reintroduced back into the patient for immunotherapeutic purposes.

In another embodiment, once PBMCs are isolated, these cells can be further isolated to provide a more homogeneous or enriched culture of CD8+ lymphocytes, and these cells then readministered to the patient.

Alternatively, the stimulated lymphocytes CD8+ obtained in step c) of the method described above may be co-cultured with treated and irradiated cells or tissue obtained from step b) above as many times as convenient in order to obtain sufficient high numbers of stimulated lymphocytes CD8+ for use in immunotherapy of the patient in question.

The present invention provides an *in vitro* method of preparing stimulated lymphocytes CD8+ for use in adoptive immunotherapy comprising isolating epithelial cells from a patient and treating them with an effective dose of OSM, a OSMR agonist, or a combination of OSM or a OSMR agonist with a IFN type I, or the recombinant expression vector of the present invention; irradiating the treated cells; co-culturing the treated and irradiated material from the patient with enriched CD8+ lymphocytes and optionally antigen presenting cells (APCs), such as autologous dendritic cells, monocytes, B cells, EBV-transformed B cell lines, allogeneic EBV transformed B cell lines expressing the shared restricting allele, artificial antigen presenting cells; and expanding these cells in culture. These expanded cells that comprise stimulated lymphocytes CD8+ may then be reintroduced back into the patient.

The present invention further relates to stimulated lymphocytes CD8+ obtainable by the methods described herein, as well as to the use of these stimulated lymphocytes CD8+ as a medicament, or in the manufacture of a medicament for the treatment of cancer or infectious diseases. Equivalently, the present invention provides a method of treating cancer or an infectious disease in a human said method comprising the administration of an effective amount of stimulated lymphocytes CD8+ obtainable according to the methods described herein.

OSM, a OSMR agonist, or a combination of OSM or a OSMR agonist with a type I interferon are therefore useful as an in vitro enhancer of immunostimulatory activity of human epithelial cells. In a particular embodiment of the invention, the type I interferon is a IFN-α whereas in another particular embodiment is a IFNβ.

The present invention is illustrated by the following examples, which are not to be construed as limiting.

### Examples

To analyze immunostimulating properties of OSM on target cells, cytokine-treated human hepatoma cells (Hih7 and HepG2) pulsed with a peptide antigen were used to stimulate antigen-specific cytotoxic T lymphocytes. Activation of lymphocytes was measured by analyzing their production of IFN-γ, a commonly used marker of T lymphocyte priming. Production of this Th1 cytokine after antigen recognition by T cells has been traditionally considered as a read-out, which correlates with effector properties of lymphocytes useful in immunotherapeutic strategies in viral infections and cancer (Brosterhus, et al., 1999).

### Material and Methods

### RNA extraction and Real Time RT-PCR

Total RNA extraction was performed using the Nucleic Acid Purification Lysis Solution (Applied BioSystems) and the semi-automated system ABI PRISM 6100 Nucleic Acid PrepStation (Applied BioSystems). Real time RT-PCR was performed as described (Larrea, et al., 2006) using specific primers for each gene (Table 1).

### Western blot

For Western Blot analysis, 1.5x10⁴ Huh7 or HepG2 cells were seeded onto 6-well plates. After 24 h, cells were left untreated or treated with 50 IU/ml of IFNα2 (Sicor) or with 20 ng/ml OSM (R&D Systems) or with 50 IU/ml IFNα2 plus 20 ng/ml OSM. After different times points, cells were PBS-washed and collected in 150 µl of protein loading buffer (62.5 mM Tris-HCl pH 6.8, 10% glycerol, 5% 2-ME, 2% SDS, 0.006% bromophenol blue). Western blot was performed (Larrea, et al., 2006) using specific antibodies: Anti-phospho-STAT1tyr701, anti-phospho-STAT3tyr705, anti-phospho-JAK1tyr1022/1023, anti-phospho-Tyk2tyr1054/1055, anti-phospho-p38thr180/tyr182 MAPK antibodies and anti-Rabbit IgG Peroxidase (HRP)-linked antibody were purchased from Cell Signaling Technology. Anti-STAT3, anti-Tyk2, anti-STAT2, anti-phospho-STAT2tyr689 antibodies were from Upstate Biotechnology. Anti-STAT1 and anti-p38 MAPK antibodies were from Santa Cruz Biotechnology. Anti-Tap1, anti-ICAM-1, anti-PSMB9, anti B2M and anti-HLA class I antibodies were from Abcam. Anti-actin and anti-mouse IgG HRP-linked antibodies were from Sigma-Aldrich.

### Microarray analysis

Huh7 cells were seeded at 1x10⁶ cells/plate in DMEM plus 10% FBS. After 18 h, cells were left untreated or treated with IFNα2 (50 IU/ml), or OSM (20 ng/ml) or IFNα2 (50 IU/ml) combined with OSM (20 ng/ml). Three days later, cells were harvested in 1 ml of TRIzol reagent (Invitrogen). The experiments were performed in quadruplicate. Samples were then processed at Progenika Biopharma S.A following Affymetrix recommendations and cRNA was hybridized to the Affymetrix human U133A 2.0 array. Both background correction and normalization were done using Robust Multichip Average algorithm. After calculation of the expression for each probeset in all the microarrays, a filtering process was performed to eliminate low expression probesets. Applying the criteria of an expression value greater than 16 in the 17% of the samples, 17,927 probesets were selected for the statistical analysis. Linear Models for Microarray Data was used to find out the probesets that showed significant differential expression between experimental conditions. Genes affected by IFNα2 or OSM or the combination of IFNα2 plus OSM treatments, were identified as significant using a B statistic cut off (B>0). Described genes were selected based on a fold change criteria and an enrichment of functional categories, studied by Ingenuity (http://www.ingenuity.com/) and Webgestalt (http://bioinfo.vanderbilt.edu/webgestalt) softwares.

### Antigen presentation assay

PBMC obtained from a HLA-A2+ healthy donor, were pulsed with 1 µg/ml of HLA-A2-restricted influenza A virus matrix58-66 peptide (GILGFVFTL) [SEQ ID NO: 46] for 2 hours at 37°C, washed and cultured into 24-well plates at density of 3×10⁶ cells/well. Three days later, IL-2 (10 U/ml) was added and cells were cultured for additional 5 days. On day 8, recovered cells were co-cultured (10⁵/well) with HepG2 cells (5x10⁴/well) previously treated for 4 days with 50 IU/ml of IFNα2 or 20 ng/ml of OSM or 50 IU/ml of IFNα2 plus 20 ng/ml of OSM or untreated cells, in presence or absence of 1 µg/ml of GILGFVFTL (SEQ ID NO: 46) peptide in 96-well round-bottom culture plates. After 24 h, the supernatants were collected to measure IFN-γ production by ELISA (BD Biosciences).

### IL-15Rα activity assay

The functionality of IL-15Rα expressed on Huh7 cells to transpresent IL-15 to CTLL-2 cells was estimated. To this aim, Huh7 cells were seeded and treated with 50 IU/ml of IFNα2, 20 ng/ml of OSM or combination of both treatments during three days. Then, Huh7 cells were harvested and incubated one hour with or without 50 ng/ml of exogenous IL-15, washed three times and irradiated at 15000 cGys in a Gammacell 3000 Elan. Then, 3x10⁴ irradiated Huh7 cells were co-cultured with 1x10⁴ CTLL-2 cells in 96-well plates. On day 2, cells were pulsed with 0.5 µCi/well of tritiated thymidine for 8 h, harvested and thymidine incorporation was measured in a scintillation counter (Topcount, Packard).

### Flow cytometry analysis

The expression of the surface molecule ICAM 1 was studied in Huh7 and HepG2 cells left untreated or treated with 50 IU/ml of IFNα2 or 20 ng/ml of OSM or 50 IU/ml IFNα2 plus 20 ng/ml of OSM during four days. Then, cells were PBS-EDTA recovered and stained with PE-labeled anti-human ICAM1 or IgG-PE control isotype (BD Biosciences). After 30 min, cells were washed and analyzed using a FACSCalibur cytometer (Becton Dickinson).

### Statistical analysis

Statistical methods used were as described previously (Larrea, et al., 2006). Data was means ± SD; a p value of <0.05 was considered significant.

**Table 1. Primers used in this study.**

| Gene | Sense primer (5'-3') | SEQ ID NO: | Antisense primer (5'-3') | SEQ ID NO: |
|---|---|---|---|---|
| B2M | AGGCTATCCAGCGTACTCCA | 2 | ATCTTTTTCAGTGGGGGTGA | 3 |
| Cxcl1/2 | CACCCCAAGAACATCCAAAG | 4 | AGCCACCAGTGAGCTTCCTC | 5 |
| Cxcl3 | CAAACCGAAGTCATAGCCAC | 6 | TTCTCTCCTGTCAGTTGGTG | 7 |
| GBP2 | TGGCTCCAGAGATCAACTTG | 8 | TTCCCTTGGTGTGAGACTTC | 9 |
| HLA-A | GATAATGTATGGCTGCGACG | 10 | GTGGGTCATATGTGTCTTGG | 11 |
| HLA-B | ACCAAACTCAGGACACTGAG | 12 | ATGACCACAACTGCTAGGAC | 13 |
| HLA-C | AGACACAGAAGTACAAGCGC | 14 | TGTAATCCTTGCCGTCGTAG | 15 |
| IL-32 | CAGTGGCGGCTTATTATGAG | 16 | GTTGCGGGATCCTCAACATC | 17 |
| IL-7 | CCCTGATCCTTGTTCTGTTG | 18 | AAATTGCCTCAACTTGCGAG | 19 |
| IL-15Rα | GGAACCACAGAGATAAGCAG | 20 | CCTTGACTTGAGGTAGCATG | 21 |
| IRF1 | CTGATACCTTCTCTGATGG | 22 | TCCAGGTTCATTGAGTAGG | 23 |
| ICAM-1 | CCGAGCTCAAGTGTCTAAAG | 24 | CCTTTTTGGGCCTGTTGTAG | 25 |
| MYD88 | TTGAGTACTTGGAGATCCGG | 26 | ACCTGTAAAGGCTTCTCAGC | 27 |
| PSMB8 | CAGAATTCTTCCAGTCCCTG | 28 | AGTACAGCCTGCATTCCTTG | 30 |
| PSMB9 | CATCATGGCAGTGGAGTTTG | 30 | TGAGATGTGCAGACAAGTCC | 31 |
| S100A9 | CTTGCAAAATGTCGCAGCTG | 32 | CATGATGAACTCCTCGAAGC | 33 |
| TAPBP | TGATGGTCAGCATATCCAGC | 34 | AAGTCCAGCAGAGCATCTTG | 35 |
| TAP1 | TCGTTGTCAGTTATGCAGCG | 36 | CCCGTAAAGAATGGAATGGC | 37 |
| TAP2 | GGACCAGGTGAACAACAAAG | 38 | CAAAGGAGAAGAGGCACATG | 39 |
| UBE2L6 | ACAACCAAGATCTACCACCC | 40 | ATTCTGTGTCAGCAGGTCAG | 41 |
| ULBP2 | GCTACCAAGATCCTTCTGTG | 42 | AGTCTTTTCATCCACCTGGC | 43 |
| □-actin | AGCCTCGCCTTTGCCGA | 44 | CTGGTGCCTGGGGCG | 45 |

### Preparation of PBMCs

Large amounts of patient PBMCs are obtained by a leukapheresis that is performed with a Spectra Cobe (Lakewood Co., USA) device employing the MNC program (semi-automated method) and processing at least one blood volume.

### In vitro stimulation of lymphocytes CD8+

The tumoral human-derived epithelial cells or tissue are obtained from a patient by surgery. The tissue extracted from the human body is processed to eliminate fat and necrotic tissue. A single-cell suspension is prepared by the well-known methods in the art such as mechanical disaggregation or sequential enzymatic digestion.

The tumoral epithelial cells are treated during 4 days with OSM, an OSM agonist, or a combination of OSM or an OSM agonist with an IFN type I.

The treated tumoral epithelial cells (TTEC) are seeded at 0.5-1 x 10⁶ cells / mL in CellGro SCGM (Cell Genix, Germany), supplemented with 10 % fetal bovine serum (FBS), and cultured at 37°C and 5% CO₂ over a 2-week period. Non-viable TTECs are removed at each change of medium (every other day). When TTECs cover at least 70% of the growth surface, and a suitable amount of TTECs is available, a negative selection with anti-fibroblast microbeads (Mini-Macs, Miltenyi Biotec, USA) is performed, according to the manufacturer's instructions. When neoplastic effusion is available, the sample is centrifuged, and TTECs obtained by negative selection are seeded into culture flasks at 1-5 x 10⁶ cells/mL in CellGro SCGM and cultured as described above. At early passages and periodically during subculture, TTECs are characterized extensively by a number of evaluation criteria (i.e. morphologic and phenotypical analyses and gene rearrangement) to address the correlation with the tissue of origin.

Early-passage, rather than long-term passages, TTECs are used to minimize the possibility of modifying primary characteristics as a consequence of extensive in vitro reculturing. TTEC lines, after morphologic and phenotypical analysis, are cryopreserved until use as stimulatory and target cells (purity > 98%).

CD8-enriched cells (10⁶ cells/mL) are co-cultured in 48-well plates (final volume 1 mL) with dendritic cells (2x10⁵ cells/mL), irradiated (20 000 rads) and TTECs (0.5-1 x 10⁵ cells/mL). After 7-10 days, cultures are restimulated with irradiated TTECs (5 x 10⁵ cells/mL). The same protocol is used for an additional round of tumor-specific stimulation.

### Results

### Jak/STAT signaling in Huh7 cells treated with IFNα and/or OSM

To analyze cell signaling mechanisms activated by the combined effect of OSM and IFNα, we performed immunoblotting analysis of Jak/STAT proteins in Huh7 cells treated for 1, 3, 24, 48 and 72 h, with IFNα2 or OSM or both. As shown in Fig. 1, STAT2 was only activated by IFNα2 or by its combination with OSM being the activation transient and not detectable by 24 h. Similarly STAT1 was strongly phosphorylated by IFNα2 at 1 and 3 h but its activation was no longer present at 24 h. However IFNα2 caused an increase of total STAT1 protein which was apparent from 24 h onwards. OSM activated STAT1 at 1 h and the signal was faint during the following time points but lasted until 72 h. OSM also increased, albeit moderately, the levels of total STAT1 protein. When IFNα2 was combined with OSM we observed an additive effect of the two cytokines resulting in increased levels of total STAT1 and prolonged activation of this molecule leading to strong activation signal of STAT1 lasting up to 72 h. Relating STAT3, IFNα2 caused only a mild and transient activation of the molecule which was no longer detectable after 1 h. In contrast OSM alone and the combination OSM plus IFNα2 induced rapid and very robust activation of STAT3 that persisted at 72 h. This was accompanied by increased levels of STAT3 protein from 24 h onwards. Moreover, OSM, alone or in combination with IFNα2, caused stronger and more prolonged activation of Jak1 than when using IFNα2 alone (Fig. 1 A).

From these findings it seems possible that the longer and stronger activation of Jak1, STAT1 and STAT3 when using OSM plus IFNα2 might facilitate durable formation of STAT1 and STAT3 homodimers and heterodimers.

Since activation of p38 MAPK has been shown to facilitate IFNα-driven gene expression through ISRE and GAS elements (Parmar, 2003) we also analyzed the effect of the two cytokines in the activation of this signaling molecule. We found that in Huh7 cells IFNα failed to induce p38 phosphorylation while OSM plus/minus IFNα caused marked p38 activation for at least 72 h (Fig. 1 B). It seems possible that this effect of OSM on p38 might contribute to enhance the expression of IFNα-sensitive genes when the two cytokines are used in combination.

### Microarray analysis of genes induced by IFNα and/or OSM

To gain insight into the transcriptional program set into motion by the joint action of IFNα2 plus OSM we studied the transcriptome of Huh7 cells incubated for 72 h in basal medium or in the presence of IFNα2 (50 IU/ml), or OSM (20 ng/ml) or both. After functional analysis studies with the genes differently expressed we found an enrichment of some biological categories that included antiviral genes, genes involved in antigen presentation and genes encoding key immunoregulatory factors.

Validation of these genes was performed by quantitative RT-PCR after RNA extraction from Huh7 cells treated with IFNα2, OSM or both for 24, 48, and 72 h. Validated genes could be grouped into two clusters: A) genes sensitive or not to IFNα which showed little or no change with OSM alone but manifested vigorous upregulation by the combination treatment; B) genes that were induced by OSM as well as by the combination of the two cytokines. Cluster A comprised mainly antiviral genes and genes implicated in antigen processing and presentation. Cluster B included genes encoding molecules relevant to innate immunity, genes implicated in lymphocyte activation and expansion as well as specific antiviral genes and genes involved in antigen presentation.

### OSM induces key players of innate immunity

Data from microarray and RT-PCR validation studies indicated that OSM participates in innate immunity not only by enhancing the expression of IFNα-stimulated antiviral genes, but also by inducing directly molecules that are essential in the natural defense against infection including MYD88, S100A9, ULBP2, IL-32, IRF1 and GBP2 and the chemokine genes CXCL1, CXCL2 and CXCL3 (Fig. 2). For several of these genes the combination OSM plus IFNα augmented their expression at levels higher than with OSM alone.

### Upregulation of molecules involved in antigen processing and presentation by combined effect of OSM and IFNα or by OSM alone

As previously indicated, a group of genes encoding molecules with essential functions in antigen processing and presentation were strongly upregulated when Huh7 cells were treated with OSM plus IFNα2. These genes included: a) members of the ubiquitin-immunoproteasome system UBE2L6, PSMB8 and PSMB9, implicated in the generation of peptides from cytosolic proteins, b) transporters of peptides into the endoplasmic reticulum for association with MHC class I molecules, namely TAP1 and TAP2, c) HLA class I genes specially HLA-A, HLA-B and HLA-C, and B2M (Fig 3 A-I) which encodes β2-microglobulin, an essential molecule for stable expression of class I molecules on cell surfaces (Rizvi, et al., 2006).

On the other hand we found that OSM per se induced other genes which are critical for antigen presentation such as TAPBP (Fig. 3 J) whose gene product mediates the interaction between TAP1 and HLA class I (Rizvi, et al., 2006).

Western blot analysis of PSMB9 and TAP1 in Huh7 cells demonstrated that treatment with IFNα2 plus OSM strongly induced the expression of these molecules at day 3 and 4 of incubation while each cytokine alone caused only a mild elevation of the same proteins (Fig. 3 K). HLA class I proteins experienced upregulation mainly with IFNα2 and less with OSM with an additive effect of the two cytokines that was apparent at day 3 but not at later time points (Fig. 3 K). B2M protein showed upregulation mainly by IFNα2 at day 3 and by the combined treatment at day 4 (Fig. 3 K).

These results indicate that the concerted action of IFNα2 and OSM on liver epithelial cells strongly stimulates the machinery responsible for the generation and presentation of antigenic peptides to the effectors of the adaptive immune response. This effect is of considerable importance to facilitate immune clearance of virus-infected cells.

### OSM increases the immunostimulatory function of Huh7 cells and their ability to transpresent IL-15

In addition to the above findings, we observed that OSM induces in Huh7 cells genes encoding molecules favoring activation and expansion of lymphocytes, namely ICAM-1, IL-15Rα, and IL-7 (Fig. 4, A-C). Western blot analysis indicated that OSM alone or in combination with IFNα2 caused upregulation of ICAM-1 with a pattern of multiple bands consistent with hyperglycosilation (Fig. 4 D), an effect associated with higher immunostimulatory activity of the protein (Diamond, et al., 1991). In agreement with these results, flow cytometry studies showed that OSM and the combined treatment with IFNα2 increased ICAM-1 abundance at the cell membrane of Huh7 cells (Fig. 4 E). Another relevant molecule conferring immunostimulatory properties to epithelial cells is IL-15Rα, which is essential for efficient transpresentation of IL-15 to CD8+ T cells. To ascertain the role of OSM in boosting the expression of functional IL-15Rα we studied the effect of OSM, IFNα2 or OSM plus IFNα2 on the ability of IL-15-pulsed Huh7 cells to sustain the proliferation of CTLL-2 cells (an IL-15 responsive cell line). As depicted in Fig. 4 F we found that OSM alone or in combination with IFNα2, caused significant stimulation of CTLL-2 proliferation, while cell growth was similar with all forms of treatment in the absence of IL-15. Our data show that OSM enhances IL-15 transpresentation by hepatocytic cells and that this effect is more potent than that exhibited by IFNα.

We further investigated whether OSM alone or in combination with IFNα2 was able to increase the immunostimulatory activity of liver epithelial cells. To this aim we incubated HepG2 cells with medium containing OSM, or IFNα2 or in combination or in medium alone and cells were then pulsed with an influenza A virus peptide presented by HLA-A2. These cells were used to stimulate the production of IFNγ by sensitized CTL. In this experiment we used HepG2 cells since they are HLA-A2+ and were shown to respond to OSM with upregulation of genes involved in antigen presentation and immunostimulation in the same manner as Huh7 cells (Fig. 4H-N). In agreement with the above data we found that pretreatment with OSM or the combination OSM plus IFNα2 enhanced the ability of HepG2 cells to stimulate the production of IFNγ by CTL more efficiently than when using IFNα2 alone (Fig. 4 G).

The positive interaction of OSM with type I IFN in the induction of immunoregulatory molecules was observed not only with IFNα but also with IFNβ (Fig. 5).

### REFERENCES

Auernhammer C J [et al.] The oncostatin M receptor/gp130 ligand murine oncostatin M induces apoptosis in adrenocortical Y-1 tumor cells // J Endocrinol.- 2004.- 3: Vol. 180.- pp. 479-86.
Blais Marie-Ève T-Cell Development: An Extrathymic Perspective.- 2006.- Vol. 209.- pp. 103-14.
Brosterhus H [et al.] Enrichment and detection of live antigen-specific CD4(+) and CD8(+) T cells based on cytokine secretion.- Eur J Immunol. 1999.- 12: Vol. 29.-pp. 4053-9.
Brown T J [et al.] Regulation of IL-6 expression by oncostatin M // J Immunol.- 1991.- 7: Vol. 147.- pp. 2175-80.
Brown T J, Lioubin M N en Marquardt H Purification and characterization of cytostatic lymphokines produced by activated human T lymphocytes. Synergistic antiproliferative activity of transforming growth factor beta 1, interferon-gamma, and oncostatin M for human melanoma cells // J Immunol.. - 1987. - 9 : Vol. 139. - pp. 2977-83.
Bruce A G en Rose T M Oncostatin // Cytokine reference.- J. Oppenheim and M. Feldmann, 2000.
Castelruiz Y [et al.] Interferon alfa subtypes and levels of type I interferons in the liver and peripheral mononuclear cells in patients with chronic hepatitis C and controls // Hepatology. - 1999. - Vol. 29. - pp. 1900-1904. Chou T C en Talalay P Quantitative analysis of dose-effect relationships: the combined effects of multiple drugs or enzyme inhibitors // Adv Enzyme Regul. - 1984. - Vol. 22. - pp. 27-55.
Diamond M S [et al.] Binding of the integrin Mac-1 (CD11b/CD18) to the third immunoglobulin-like domain of ICAM-1 (CD54) and its regulation by glycosylation // Cell. - 1991. - Vol. 65. - pp. 961-971.
Durda Paul J [et al.] Induction of "Antigen Silencing" in Melanomas by Oncostatin M: Down-Modulation of Melanocyte Antigen Expression // Molecular Cancer Research. - 2003. - Vol. 1. - pp. 411-9.
Gibbs P, Chen Q en Robinson W A Effects of oncostatin M and tamoxifen on human melanoma cells // Melanoma Res.. - 1998. - Vol. 8. - pp. 221 - 226.
Grant S L en Begley C G The oncostatin M signalling pathway: reversing the neoplastic phenotype? // Mol Med Today. - 1999. - 9 : Vol. 5. - pp. 406-12.
Hams Emily [et al.] Oncostatin M Receptor-Beta Signaling Limits Monocytic Cell Recruitment in Acute Inflammation." // Journal of Immunology. - 2008. - pp. 2174-80.
Heinrich P C Interleukin-6 and related cytokines: effect on the acute phase reaction // Z Ernahrungswiss. - 1998. - Suppl 1 : Vol. 37. - pp. 43-9.
Hung M-C Nonviral Vectors for Gene Therapy: Academic Press, 1999.
Ichihara M Oncostatin M and leukemia inhibitory factor do not use the same functional receptor in mice // Blood. - 1997. - 1 : Vol. 90. - pp. 165-73.
Kramer M G [et al.] In vitro and in vivo comparative study of chimeric liver-specific promoters // Molecular Therapy. - 2003. - 3 : Vol. 7. - pp. 375-85.
Larrea E [et al.] Altered expression and activation of signal transducers and activators of transcription (STATs) in hepatitis C virus infection: in vivo and in vitro studies // Gut. - 2006. - Vol. 55. - pp. 1188-1196.
Larrea E [et al.] IFN-alpha5 mediates stronger Tyk2-stat-dependent activation and higher expression of 2',5'-oligoadenylate synthetase than IFN-alpha2 in liver cells. // Interferon Cytokine Res. - 2004. - Vol. 24. - pp. 497-503.
Le Bon A [et al.] Cross-priming of CD8+ T cells stimulated by virus-induced type I interferon // Nat Immunol. - 2003. - 10 : Vol. 4. - pp. 1009-15.
Le Bon A Durand V, Kamphuis E, Thompson C, Bulfone-Paus S, Rossmann C, Kalinke U, Tough DF Direct stimulation of T cells by type I IFN enhances the CD8+ T cell response during cross-priming // J Immunol. - 2006. - 8 : Vol. 176. - pp. 4682-9.
Le Bon A Thompson C, Kamphuis E, Durand V, Rossmann C, Kalinke U, Tough DF Cutting edge: enhancement of antibody responses through direct stimulation of B and T cells by type I IFN // J Immunol. - 2006. - 4 : Vol. 176. - pp. 2074-8.
Malik N Molecular cloning, sequence analysis, and functional expression of a novel growth regulator, oncostatin // M. Mol Cell Biol. - 1989. - 7 : Vol. 9. - pp. 2847-53.
Mather Jennie P: WO2006/084092. - 2006.
Miles S A [et al.] Oncostatin M as a potent mitogen for AIDS-Kaposi's sarcoma-derived cells// Science. - 1992. - Vol. 255. - pp. 1432-1434.
Miller J B [et al.] Cellular and molecular diversity in skeletal muscle development: news from in vitro and in vivo // Bioessays. - 1993. - 3 : Vol. 15. - pp. 191-6.
Modur V [et al.] Oncostatin M is a proinflammatory mediator: in vivo effects correlate with endothelial cell expression of inflammatory cytokines and adhesion molecules // J. Clin. Invest.. - 1997. - Vol. 100. - pp. 158-168.
Murata M Nabeshima S, Kikuchi K, Yamaji K, Furusyo N, Hayashi J A comparison of the antitumor effects of interferon-alpha and beta on human hepatocellular carcinoma cell lines - Cytokine 2006. - 3 : Vol. 33. - pp. 121-8.
Nair B C [et al.] Identification of a major growth factor for AIDS-Kaposi's sarcoma cells as oncostatin M // Science. - 1992. - Vol. 255. - pp. 1430-1432.
Nishimoto N, Ogata A and Shima Y Oncostatin M, leukemia inhibitory factor, and interleuki n 6 induce the proliferation of human plasmacytoma cells via the common signal transducer, gp130// J Exp Med.. - Apr 1, 1994. - 4 : Vol. 179. - pp. 1343-7.
Parmar S., and L.C. Platanias Interferons: mechanisms of action and clinical applications// Curr Opin Oncol. - 2003. - Vol. 15. - pp. 431-439
Pestka S, Krause C D en Walter M R Interferons, interferon-like cytokines, and their receptors // Immunol Rev. - 2004. - Vol. 202. - pp. 8-32.
Pietschmann T [et al.] Persistent and transient replication of full-length hepatitis C virus genomes in cell culture // J Virol. - 2002. - Vol. 76. - pp. 4008-4021.
Rizvi S M en Raghavan M Direct peptide-regulatable interactions between MHC class I molecules and tapasin // Proc Natl Acad Sci. - 2006. - Vol. 103. - pp. 18220-18225.
Rose T M en Bruce A G Oncostatin M is a member of a cytokine family that includes leukemia-inhibitory factor, granulocyte colony-stimulating factor, and interleukin 6 // Proc. Nat. Acad. Sci.. - 1991. - Vol. 88. - pp. 8641-8645.
Sambrook J en Russell D W Molecular cloning : a laboratory manual: Cold Spring Harbor Laboratory, 2001. - 3rd.
Sarobe P [et al.] Abnormal priming of CD4(+) T cells by dendritic cells expressing hepatitis C virus core and E1 proteins // J Virol. - 2002. - Vol. 76. - pp. 5062-5070.
Suda T [et al.] Oncostatin M production by human dendritic cells in response to bacterial products // Cytokine. - 2002. - 6 : Vol. 17. - pp. 335-40.
Tanaka M en Miyajima A Oncostatin M, a multifunctional cytokine // Rev Physiol Biochem Pharmacol.. - 2003. - Vol. 149. - pp. 39-52.
Turin, I., Pedrazzoli, P., Tullio, C., Montini, E., La Grotteria, M Carmela, Schiavo, R., Perotti, C., Locatelli, F., Carretto, E., Maccario, R., Siena, S. and Montagna, D. GMP production of anti-tumor cytotoxic T-cell lines for adoptive T-cell therapy in patients with solid neoplasia // Cytotherapy - 2007 - Vol. 9:5 - pp. 499 - 507
Wallace Philip M [et al.] Regulation of Inflammatory Responses by Oncostatin M // The Journal of Immunology. - 1999. - Vol. 162. - pp. 5547-5555.
Yao L Interleukin 4 or oncostatin M induces a prolonged increase in P-selectin mRNA and protein in human endothelial cells // J Exp Med. - 1996. - 1 : Vol. 184. - pp. 81-92.
Zarling J M [et al.] Oncostatin M: a growth regulator produced by differentiated histiocytic lymphoma cells // Proc. Nat. Acad. Sci.. - 1986. - Vol. 83. - pp. 9739-9743.

## Claims

1. Use of oncostatin M (OSM), a OSM receptor (OSMR) agonist, or a combination of OSM or a OSMR agonist with an interferon type I (IFN type I); in the manufacture of a medicament for enhancing the immunostimulatory activity of epithelial cells in a human.

2. The use according to claim 1, wherein the immune response is an adaptive immune response.

3. The use according to any one of claims 1-2, wherein the immunostimulatory activity of epithelial cells comprises the proliferation of T lymphocytes, the transpresentation of interleukin 15 (IL-15) to NK or CD8+ cells, or the production of interferon gamma (IFN-γ).

4. The use according to any one of claims 1-3, wherein the epithelial cells are infected with a cellular pathogen or are tumor epithelial cells.

5. The use according to claim 4, wherein the cellular pathogen is selected from a virus, bacteria, fungi, and parasite.

6. OSM, a OSMR agonist, or a combination of OSM or a OSMR agonist with IFN type I for use in enhancing the immunostimulatory activity of epithelial cells in a human.

7. A method of enhancing the immunostimulatory activity of epithelial cells in a human, comprising administering to said human an effective amount of OSM, a OSMR agonist, or a combination of OSM or a OSMR agonist with IFN type I.

8. A product comprising OSM or a OSMR agonist, and a IFN type I as a combined preparation for simultaneous, separate, or sequential use for enhancing the immunostimulatory activity of epithelial cells in a human.

9. A recombinant expression vector comprising a nucleic acid encoding OSM or a OSMR agonist, and optionally a nucleic acid encoding interferon type I, operably linked to one or more control sequences that direct the production of the OSM or OSMR agonist, and optionally a IFN type I in a suitable expression host.

10. A host cell comprising the recombinant expression vector according to claim 9.

11. The host cell according to claim 10, wherein said host cell is a prokaryotic or an eukaryotic cell.

12. Use of the recombinant expression vector according to claim 9, or a host cell according to any one of claims 10-11, in the manufacture of a medicament for enhancing the immunostimulatory activity of epithelial cells in a human.

13. A pharmaceutical formulation comprising the recombinant expression vector according to claim 9, or a host cell according to any one of claims 10-11, and a pharmaceutically acceptable excipient.

14. An *in vitro* method for the preparation of stimulated lymphocytes CD8+ for use in adoptive immunotherapy, said method comprising:
a) treating human-derived epithelial cells or tissue with an effective dose of OSM, a OSMR agonist, or a combination of OSM or a OSMR agonist with a IFN type I, or the recombinant expression vector according to claim 9;
b) irradiating the treated cells or tissue obtained from step a);
c) co-culturing the irradiated cells or tissue obtained from step b) with
• peripheral blood mononuclear cells (PBMCs), or
• enriched CD8+ lymphocytes and optionally antigen-presenting cells;
d) optionally co-culturing the stimulated CD8+ lymphocytes obtained from step c) with treated and irradiated cells or tissue obtained from step b);
wherein the PBMCs, CD8+ lymphocytes, and epithelial cells or tissue are derived from the same human subject.

15. The *in vitro* method according to claim 14, wherein the antigen-presenting cells in step c) are dendritic cells.

16. Stimulated lymphocytes CD8+ obtainable by the method according to any one of claims 14-15.

17. Use of the stimulated lymphocytes CD8+ of claim 16 as a medicament.

18. Use of the stimulated lymphocytes CD8+ of claim 16 in the manufacture of a medicament for the treatment of cancer or infectious diseases.

19. Stimulated lymphocytes CD8+ of claim 16 for use in the treatment of cancer or infectious diseases.

20. A method of treating cancer or an infectious disease in a human said method comprising the administration of an effective amount of stimulated lymphocytes CD8+ according to claim 16.

21. *In vitro* use of OSM, a OSMR agonist, or a combination of OSM or a OSMR agonist with a IFN type I, as an enhancer of immunostimulatory activity of human epithelial cells.
